(19) Europäisches Patentamt
European Patent Office
Office européen des brevets

(11) Publication number : 0 489 691 A2

(12) **EUROPEAN PATENT APPLICATION**

(21) Application number : 91810928.1

(22) Date of filing : 27.11.91

(51) Int. Cl.[5] : C07C 69/83, C08G 73/10, C08L 79/08, C08K 5/12

(30) Priority : 05.12.90 US 622871

(43) Date of publication of application : 10.06.92 Bulletin 92/24

(84) Designated Contracting States : DE ES FR GB IT NL SE

(71) Applicant : CIBA-GEIGY AG
Klybeckstrasse 141
CH-4002 Basel (CH)

(72) Inventor : Blyakhman, Yefim
4705 Henry Hudson Parkway
Bronx, NY 10471 (US)

# (54) Tetraallylesters as coreactants for bismaleimides.

(57) Tetraallylesters of the formula (I)

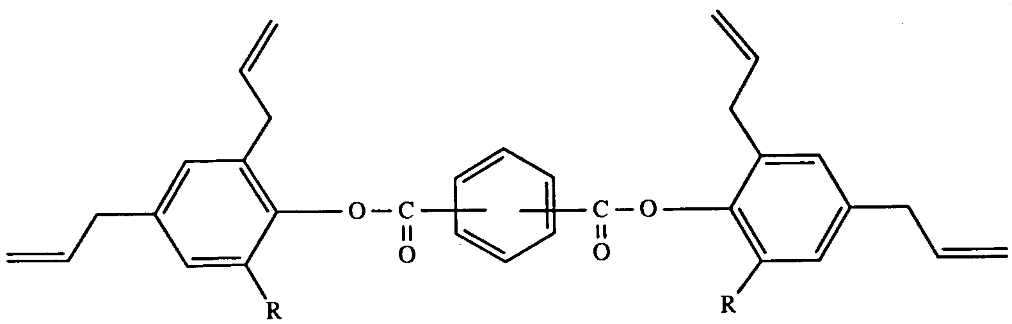

(I),

wherein R is hydrogen, $C_1$-$C_4$alkyl or -$OCH_3$ are useful as coreactants for bismaleimides. Compositions comprising bismaleimides and the tetraallylesters of the present invention may be useful as curable coatings, castings, adhesive and impregnating resins.

EP 0 489 691 A2

The present invention relates to tetraallylesters as coreactants for bismaleimides and to curable compositions comprising these tetraallylesters.

Background of the Invention

Addition type thermoset polyimide resins are finding increased use as matrix resins for advanced composites, adhesives and coating applications. Many different approaches have been explored to obtain resins and composites which are easy to process but better in mechanical strength and temperature resistance. The fastest growing area of addition-type polyimides is that of bismaleimides (BMI) which are synthesized by condensation of maleic anhydride with aromatic diamines and subsequent imidization. The most commonly used building block in BMI chemistry is bis(4-maleimidophenyl)methane:

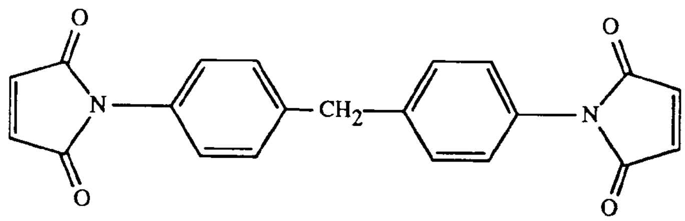

When homopolymerized, highly crosslinked and highly aromatic backbones result producing a very thermally stable but brittle matrix. Considerable formulation work has been carried out to improve the toughness and processability while retaining high temperature properties. To achieve these goals, bismaleimides have been blended and coreacted with allyl/vinyl monomers (Stenzenberger et al, 29th Nat. SAMPE, p. 1043 (1985)), thermo-plastics (Yamamoto et al, 30th Nat. SAMPE, p. 903 (1985)) and reactive rubbers (Shaw et al, Int. J. of Adhesion and Adhesives, Vol. 5, p. 123 (1985)). The best results have been obtained with allyl phenyl type coreactants (Stenzenberger, British Polymer J., Vol. 20, p. 383 (1988)). However, a coreactant which improves thermal properties, i.e. increased modulus retention at high temperatures and thermo-oxidative stability of the cured polyimide resin, is needed.

Accordingly, it is a further object of the present invention to provide curable compositions comprising bismaleimides and tetraallylesters which exhibit the aforementioned properties.

Various other objects and advantages of this invention will become apparent from the following description thereof.

Summary of the Invention

Tetraallylesters of the formula (I)

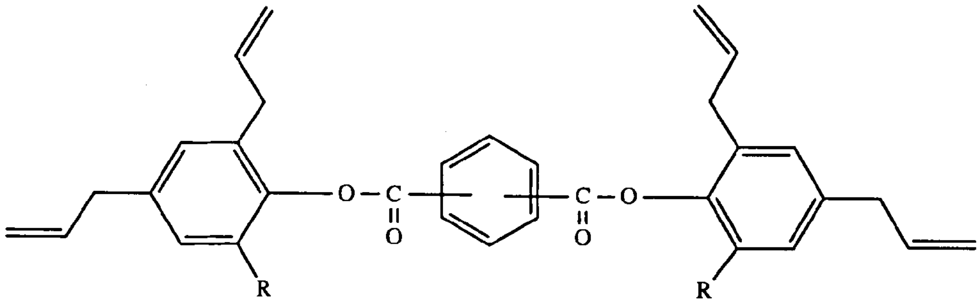

(I),

wherein R is hydrogen, $C_1$-$C_4$alkyl or $-OCH_3$ are useful as coreactants for bismaleimides. Compositions comprising bismaleimides and the tetraallylesters of the present invention may be useful as curable coatings, castings, adhesive and impregnating resins.

Detailed Description of the Invention

The present invention relates to tetraallylesters of the formula (I)

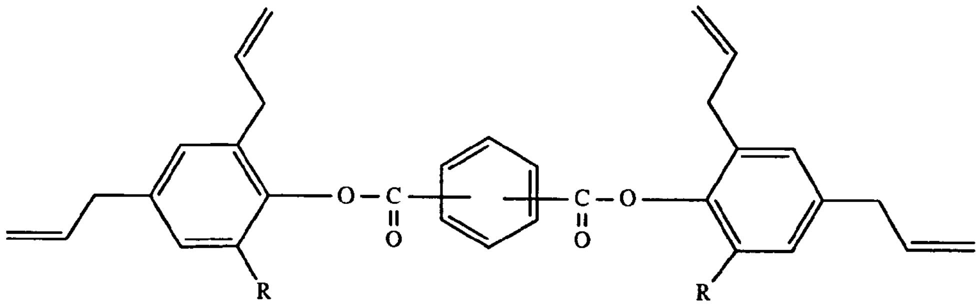

(I),

wherein R is hydrogen, $C_1$-$C_4$alkyl or -$OCH_3$. Preferably R is hydrogen or -$OCH_3$, and most preferably -$OCH_3$.

The tetraallylesters of the present invention can be obtained by the reaction of allylphenols with isophthaloyl chloride as exemplified in the reaction scheme below:

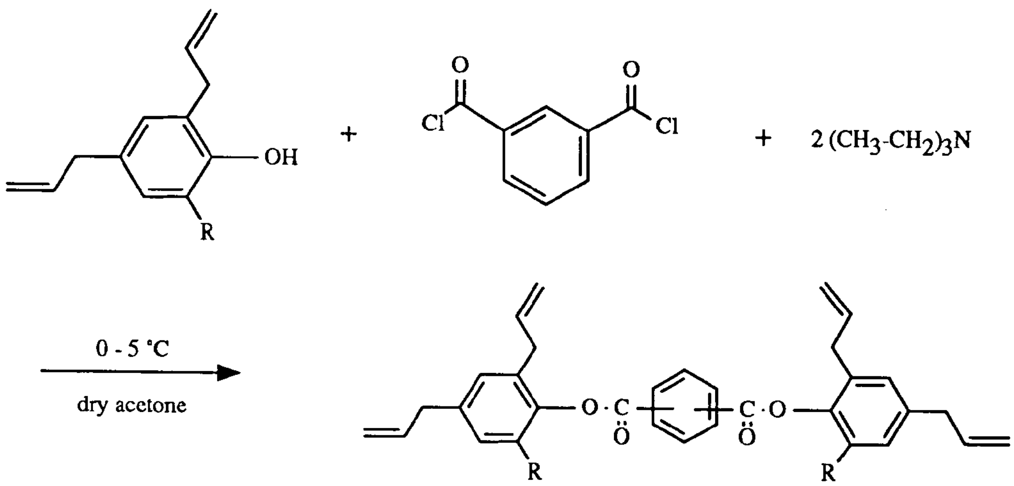


The substituted diallylphenol starting materials can be obtained by the reaction of commercially available allylphenols with allyl chloride using a variety of well-known methods as exemplified in the reaction scheme below.

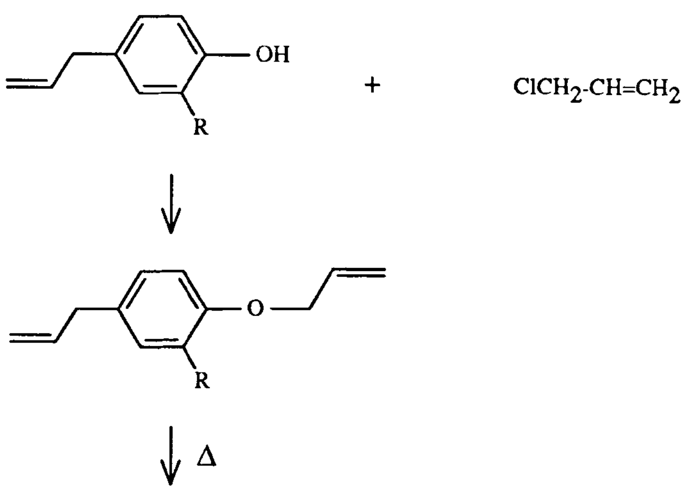

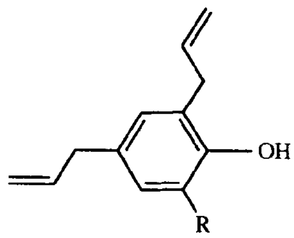

Allylphenols not readily available commercially may be obtained by well-known processes, such as from the corresponding allyl phenyl ethers by a thermal isomerization process.

The invention further relates to a bismaleimide resin composition comprising one or more bismaleimides and at least one tetraallylester of the formula (I)

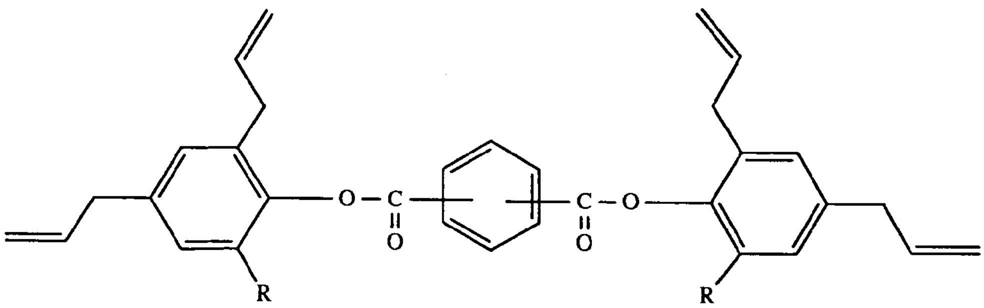

(I),

wherein R is hydrogen, $C_1$-$C_4$alkyl or $-OCH_3$.

The bismaleimides useful in the formulations of this invention may be any of the bismaleimides derived from aromatic and aliphatic diamines, including any of the well-known and widely available phenylene diamines and the various diamino-substituted polynuclear aromatic compounds such as diaminodiphenyl sulfone, diaminobenzophenone, diaminodiphenylether, diaminodiphenylmethane, and the like, as well as the various aryl compounds having a plurality of aminophenylalkylidene or aminophenoxy substituents. Also useful are bismaleimides based on $C_4$-$C_{20}$aliphatic diamines such as the various isomeric alkanes having diamino substituents. The bismaleimides may be employed singly or in mixtures comprising two or more bismaleimides, which may include both aromatic and aliphatic bismaleimides. A wide variety of bismaleimides suitable for use as matrix resins are well-known in the art, such as are recited for example in US-A 4,644,039, 4,100,140 and 4,654,407. Preferably, bis(4-maleimidophenyl) methane is employed.

The bismaleimide formulations of the present invention comprise about 50-60 parts by weight of the bismaleimide and from about 1 to about 50 parts, preferably 40 to about 45 parts, by weight of the tetraallylester. The formulations will be readily prepared by simple mixing operations ordinarily employed in the resin formulating art and may, if desired be compounded at moderately elevated temperatures to reduce the viscosity of the mixture.

The formulations may further include from 1 to about 30 % by weight, based on total resin formulation, of a thermoplastic polymer such as, for example, a polyaryl ether, a polyaryl sulfone, a polyarylate, a polyamide, a polyaryl ketone, a polyimide, a polyimide-ether, a polyolefin, an ABS resin, a polydiene or diene copolymer, polycarbonate or a mixture thereof.

The formulations of the present invention may further include from 1 about 10 % by weight, based on total resin formulation, of reactive diluents and modifiers ordinarily employed in bismaleimide resin compositions, such as, for example, vinylic coreactants such as N-vinyl-2-pyrrolidinone, alkylene glycol vinyl ethers, vinyl toluene, styrene, divinyl benzene and the like, acrylates and methacrylates such as ethylene glycol dimethacrylate, acrylates and methacrylates of polyols such as trimethylol propane and pentaerythritol, allylic compounds such as triallyl isocyanurate, diallyl phthalate, tetraallyl pyromellitate, o,o′-diallyl bisphenol A, eugenol, aryl allyl ethers such as the diallyl ether of bisphenol A and the like. Preferably, o,o′-diallyl bisphenol A is employed. Other coreactive modifiers may also be included in the formulations of this invention, such as, for example epoxy resins, cyanate ester resins and mixtures thereof, together with appropriate curing aids and accelerators typically employed in formulating such curable compositions.

The formulations may also include 0 to 3 % by weight of one or more initiators for vinyl polymerization such

as di-t-butyl peroxide, dicumyl peroxide, 1,1-bis(t-butylperoxy) cyclohexane, azo-bis-isobutyronitrile, t-butyl perbenzoate, and the like. Inhibitors for vinyl polymerizations, such as hydroquinone, t-butyl hydroquinone, benzoquinone, p-methoxyphenol, phenothiazine, 4-nitro-m-cresol, and the like may also be employed in amount of from 0 to 2 % by weight.

The bismaleimide formulations of the invention are particularly useful in combination with structural fiber for producing fiber reinforced laminates and composites and for the manufacture of prepreg. The structural fibers which may be used for these purposes include carbon, graphite, glass, silicon carbide, poly(benzothiazole), poly(benzimidazole), poly(benzoxazole), aluminum, titanium, boron, and aromatic polyamide fibers. These fibers are characterized by a tensile strength of greater than 700 $MN/m^2$, a tensile modulus of greater than 13,800 $MN/m^2$and a decomposition temperature of greater than 200 °C. The fibers may be used in the form of continuous tows (1000 to 400,000 filaments each), woven cloth, whiskers, chopped fiber or random mat. The preferred fibers are carbon fibers, aromatic polyamide fibers, such as Kevlar 49 fiber (obtained from E.I. DuPont Company) and silicon carbide fibers, aromatic polyamide fibers. The composites will generally comprise from about 10 to about 90 % by weight fiber, based on total weight of composite.

Preimpregnated reinforcement, or prepreg, may be made by combining the resin formulations with a structural fiber, using any of the variety of methods known in the art such as wet winding, hot melt fiber impregnation or impregnation with a solvent varnish. Tacky, drapable prepreg tape or tow can be produced having a long prepreg out time at room temperature, typically one to four weeks. Alternatively fiberous preforms introduced to closed molds may be impregnated by injection of low viscosity resin compositions into the mold followed by thermal gellation, the so-called resin transfer molding technique.

The compositions of this invention may be used as matrix resins for composites, high temperature coatings and adhesives. When reinforced with structural fibers, they may be used as aircraft parts as automotive parts such as drive shafts, bumpers and springs, and as pressure vessels, tanks and pipes. They are also suitable for use in a wide variety of sporting goods applications such as golf shafts, tennis rackets and fishing rods.

In addition to structural fibers, the composition may also contain particulate fillers such as talc, mica, calcium carbonate, aluminum trihydrate, glass microballoons, phenolic thermospheres, and carbon black. Up to half of the weight of structural fiber in the composition may be replaced by filler. Thixotropic agents such as fumed silica may also be used.

## Examples

The following examples serve to give specific illustrations of the practice of this invention but they are not intended in any way to limit the scope of this invention.

### Example 1:

Synthesis of the diester of 2,4-diallyl-6-methoxyphenol and isophthalic acid

A solution of 204 g (1.0 mole) of 2,4-diallyl-6-methoxy-phenol and 101.5 g (0.5 mole) of isophthaloyl chloride in 1500 ml of dry acetone is cooled to 0 °C and 121 g of triethylamine are added under $N_2$ with stirring. After the addition of triethylamine is completed, the reaction mixture is stirred at room temperature for 2 hours and then at 50-55 °C for 1 hour. Finally, the reaction mixture is cooled to 10 °C and filtered. Acetone is distilled off and 1 liter of toluene is added. The solution is washed with 500 ml of water three times and the toluene is distilled off. 250 g (92 % yield) of liquid resin is obtained. The structure is confirmed by IR and NMR and the content of the tetraallyl ester is 90% by HPLC.

### Example 2:

Synthesis of the diester of 2,4-diallyl-6-methoxyphenol and terephthalic acid

A solution of 102 g (0.5 mole) of 2,4-diallyl-6-methoxyphenol and 51.75 g (0.25 mole) of terephthaloyl chloride in 1 liter of dry acetone is cooled to 0 °C and 61 g of dry triethylamine is added under nitrogen with stirring. Thereafter, the reaction mixture is stirred at room temperature for 3 hours and at 50-55 °C for 1 hour. After cooling to 5 °C, the mixture is filtered, acetone is distilled off and 1 liter of toluene is added. The solution is washed three times with 500 ml of water and the toluene is distilled off. 121 g (89 % yield) of semisolid resin is obtained. The structure is confirmed by IR and NMR and the content of the tetraallyl ester is 85 % by HPLC.

Examples 3:

Bismaleimide formulations with the diester of 2,4-diallyl-6-methoxyphenol and isophthalic acid

A mixture of 2 moles of methylene-dianiline bismaleimide and one mole of the tetrallylester of Example 1 (100:77 weight ratio) is heated at 130-135 °C under vacuum for 20-25 minutes until a clear homogeneous melt is formed.

Upon cooling to ambient temperature, a semisolid exhibiting a viscosity of 220 mPa·s at 125 °C is formed. The viscosity of the formulation was measured using a Rheometrics RDA 700 Dynamic Spectrometer with oscillating parallel plates (40 mm in diameter) at 1 Hz and a sample height of 0.80 mm.

Dynamic Mechanical Analysis (DMA) is done using a DuPont 983 Dynamic Mechanical Analyzer. Sample dimensions are 12.7 mm wide × 76.0 mm long × 3.2 mm thick. Cured samples are heated from 25 to 400 °C at 10 °C/min under nitrogen. Resonance mode analysis is employed. Modules retention as a function of temperature is measured under dry and wet (48 hours in 72 °C water prior to testing) conditions. DMA data (dry and hot/wet) for the resin formulation of example 3 cured with a standard cure cycle ( 180 °C for 1 hour, 200 °C for 2 hours and 250 °C for 5 hours) are presented in Tables 1 and 2.

Example 4:

Bismaleimide formulation with the diester of 2,4-diallyl-6-methoxyphenol and terephthalic acid

A mixture of 2 moles of methylene-dianiline bismaleimide and 1 mole of the tetraallylester of Example 2 (100:77 weight ratio) is prepared, cured and tested using the procedure of Example 3. DMA data are presented in Tables 1 and 2.

Table 1: DMA Modulus Dry

| Temperature (°C) | Modulus ($MN/m^2$) | | Modulus Retention (%) | |
|---|---|---|---|---|
| | Example 3 | Example 4 | Example 3 | Example 4 |
| 30 | 3199 | 3344 | 100 | -- |
| 150 | 2475 | -- | 77 | -- |
| 200 | 2248 | 2296 | 70 | -- |
| 250 | 2124 | -- | 66 | -- |
| 300 | 1999 | 2034 | 62.5 | -- |
| 320 | 1931 | -- | 60.4 | -- |
| 350 | 1758 | 1793 | 55 | -- |
| 360 | 1675 | -- | 52.5 | -- |

Table 2: DMA Modulus Hot/Wet

| Temperature (°C) | Modulus ($MN/m^2$) | | Modulus Retention (%) | |
|---|---|---|---|---|
| | Example 3 | Example 4 | Example 3 | Example 4 |
| 30 | 3555 | -- | 100 | -- |
| 100 | 2561 | -- | 72 | -- |
| 200 | 2289 | -- | 64 | -- |
| 250 | 2158 | -- | 60.5 | -- |
| 300 | 1999 | -- | 56 | -- |

Thermo-oxidative stability is analyzed by Thermal Gravimetric Analysis (TGA; DuPont Model 951/Thermogravimetric Analyzer). Testing is conducted in air at 10 °C/min. The results are presented in Table 3.

Table 3: Thermo-oxidative Stability of the Compositions

| Example | $T_i$ (°C)[(1)] |
|---|---|
| 3 | 455 |
| 4 | 457 |

(1) Temperature corresponding to 5 % weight loss in TGA (air)

## Claims

1. A tetraallylester of the formula (I)

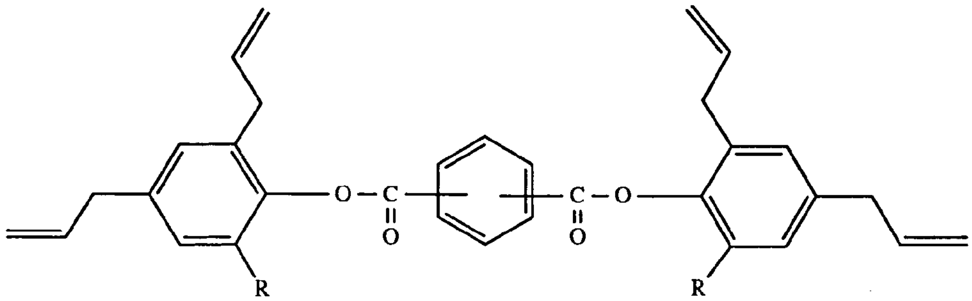

(I),

wherein R is hydrogen, $C_1$-$C_4$alkyl or $-OCH_3$.

2. A tetraallyl ester according to claim 1 wherein R is hydrogen.

3. A tetrallylester ester according to claim 1 wherein R is $-OCH_3$.

4. A curable composition comprising one or more bismaleimides and at least one tetrallylester of the formula (I)

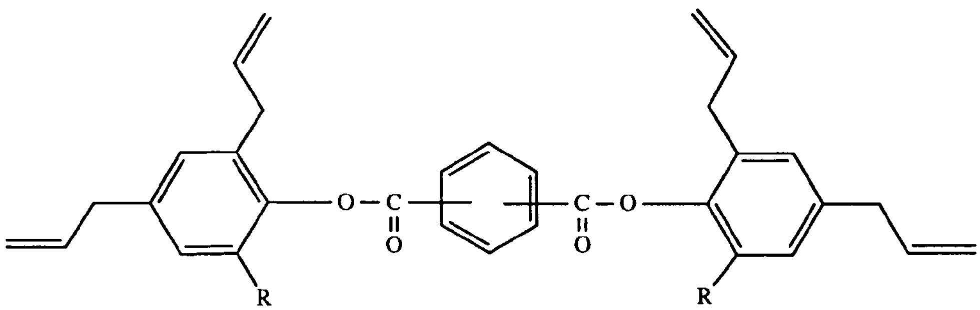

(I),

wherein R is hydrogen, $C_1$-$C_4$alkyl or $-OCH_3$.

5. A curable composition according to claim 4 wherein R of said tetrallylester is hydrogen.

6. A curable composition according to claim 4 wherein R of said tetrallylester is $-OCH_3$.

7. A curable composition according to claim 4 wherein said bismaleimide is bis (4-maleimidophenyl)methane.

8. A curable composition according to claim 4 wherein said bismaleimides are present in an amount ranging from about 50 to about 60 parts by weight.

9. A curable composition according to claim 4 wherein said tetraallylester is present in an amount ranging from about 1 to about 50 parts by weight.

10. A curable composition according to claim 4 wherein said tetraallylester is present in an amount ranging from about 40 to about 45 parts by weight.

11. A curable composition according to claim 4 further comprising a thermoplastic polymer.

12. A curable composition according to claim 4 further comprising a reactive diluents and modifiers.

13. A curable composition according to claim 4 further comprising one or more vinyl polymerization initiators.

14. A curable composition according to claim 12 wherein said reactive diluent is o,o′-diallyl bisphenol A.